# EUROPEAN PATENT APPLICATION

(11) **EP 3 798 951 A1**
(43) Date of publication of application: **31.03.2021**
(21) Application number: 20197582.8
(22) Date of filing: 22.09.2020
(51) Int. Cl.: G06Q 10/06, G06Q 50/30

(54) **METHOD AND SYSTEM FOR REDUCING THE ENVIRONMENTAL POLLUTION DUE TO A TRANSPORT ROUTE**

(30) Priority: 27.09.2019 DE 102019126207
(71) Applicant: Harman Becker Automotive Systems GmbH, 76307 Karlsbad (DE)
(72) Inventor: PREUSS, Stephan, 85247 Schwabhausen (DE); HARIHARAKRISHNAN, Anilkumar, 82054 Sauerlach (DE); ZOIDL, Richard, 85301 Schweitenkirchen (DE)
(74) Representative: Rummler, Felix

(57) **Abstract**

A method and a system for reducing the environmental pollution due to a transport route, wherein the transport route contains at least one transport means, a transport material, and a route, is characterized in that an environmental account (10) is provided, on which environmental units are managed, in that a transport route causes costs in the form of a number of environmental units and these costs are deducted from the environmental account, and in that a transport route is selected in dependence on the costs. (Figure 3)

## Description

The invention relates to a method and a system for reducing the environmental pollution due to a transport route.

A transport route can be characterized here by at least one transport means, a transport material, and a route. The transport material of a transport route can comprise in particular persons and/or products and/or goods. A route is generally defined by a start and a destination.

The goal of reducing environmental pollution as a whole exists in society and also on the part of the government. A not insignificant fraction of the environmental pollution is contributed, for example, by the traffic by motor vehicles. The individual traffic by passenger vehicles and also the product transport by trucks are included therein.

A presently disputed way of reducing the environmental pollution is to enact driving bans for specific motor vehicles in defined environmental zones.

The object of the application is to provide a method and a system by which uniform and simple reduction of the environmental pollution is made possible.

This object is achieved by a method having the features of claim 1 and a system having the features of claim 12.

The method according to the invention is characterized in that an environmental account is provided, on which environmental units are managed, that a transport route causes costs in the form of a number of environmental units and these costs are deducted from the environmental account, and that a transport route is selected in dependence on the costs.

It is clear here that the environmental pollution of a transport route is dependent on multiple factors. If a route is covered, for example, via bicycle as the transport means, the environmental pollution with identical route and identical transport material is substantially less than if a passenger vehicle is used. This means that, for example, a reduction of the environmental pollution can be achieved by suitable selection of the transport means. In the method according to the invention, fewer costs thus result, so that after the transport route is completed, fewer environmental units are deducted from the environmental account.

The advantage is that by way of the environmental account, a transparent means is available by which transport routes can be planned and/or selected on the basis of the occurring costs. The method can thus easily contribute to reducing the overall environmental pollution, wherein driving bans can be substantially dispensed with.

In one advantageous embodiment, the environmental account is assigned to a transport means and/or a user and/or a transport material. The costs can be easily assigned in this way.

In one advantageous embodiment, the transport route can be selected in dependence on the available environmental units on the environmental account, so that the environmental account does not sink below zero environmental units. A guiding function can thus result. For example, if only a limited number of environmental units is available, there is a greater incentive to select an environmentally-friendly transport route.

The selection of the transport route can in principle take place automatically, in an automated manner, or manually. In particular, the transport means and/or the route can be adapted or selected so that the lowest possible costs result for the environmental account.

In addition to the costs or the environmental pollution, other factors can also be taken into consideration in the selection of the transport route.

In one embodiment, a transport route can be selected in dependence on health data and/or fitness data of a user. The advantage is that the health of the user can also be promoted. In particular, it can be specified for short routes, for example, that the user is to ride a bicycle or to run.

In a further embodiment, a transport route can be selected in dependence on a current environmental pollution of a route. In this manner, driving bans can be prevented, since particularly strongly polluted routes are avoided and thus a distribution of the environmental pollution can be effectuated. Traffic jams can thus also be avoided and the traffic can be made more free-flowing overall.

In one embodiment, a transport route can be selected in dependence on administrative requirements. In this manner, existing or temporary bans or restrictions can be taken into consideration in the selection. Thus, for example, at specific times of day, the costs of a transport route can be higher due to administrative requirements, for example, to equalize typical commuter flows. Individual routes which are blocked, for example, by driving bans can be bypassed in that alternative routes or alternative transport means are selected. Overall, the environmental pollution can thus be reduced so that such driving bans are no longer necessary or are necessary very rarely.

The option also exists that a group of users unite, for example, via a marketplace for environmental units or via Internet portals in a guideline which is voluntary or under private law to reduce the environmental pollution, so that this can be taken into consideration in the selection of the transport route.

In dependence on various factors, it can be advantageous if a transport route is divided into multiple partial transport routes, wherein each partial transport route has at least one transport means and a route and costs linked thereto in environmental units for the environmental account. In this manner, for example, for the commute from the surrounding area into a city, it can be selected that a first partial route is covered using a passenger vehicle and a second partial route adjoining thereon is covered using the train or a suburban railway.

The division into partial routes can in turn be dependent on various factors. Thus, for example, for a transport route having the same route but having an additional transport material, such as a further person or a product, a different division or no division can be proposed, since the resulting total environmental pollution is thus less.

However, a user is free to use a transport route deviating from the proposed transport route. This means that he can use a passenger vehicle, for example, even if a train was proposed. It is accordingly expedient if an ascertainment of the costs of a transport route is carried out in dependence on the actually covered transport route. In this manner, such deviations from the proposed transport route can be taken into consideration easily.

For this purpose, it can be reasonable for route monitoring to take place, for example, by a portable device on the user and/or by a device connected to a transport means.

The costs of a transport route can be dependent on many factors. In particular, it is expedient if the costs are dependent on the transport means, on the route, on the current environmental pollution, for example, of a route, on the utilization of a transport means, and/or on the transport material. The costs can additionally be dependent on the time of day, the season, or other chronological specifications, for example, vacation times.

There is also the option of orienting the cost to measured values, for example, to a real measured fuel consumption or pollutant emission. Thus, for example, a more environmentally-friendly way of driving with otherwise identical transport route can be rewarded by lower environmental costs. Overall, an environmentally-friendly mode of transport can also be rewarded in that environmental units are credited if, for example, a transport route is covered on foot or using the bicycle. An additional incentive can thus be provided.

In one embodiment of the invention, it can be provided that environmental units can be traded, in given, loaned, or transferred in another manner. In this manner, for example, users having low environmental costs can sell their free environmental units to other users.

It can also be provided that costs are grouped or divided within a group. For example, if multiple persons form a carpool, lower environmental costs result per person, since then each person can have deductions from their environmental account.

A marketplace can expediently be provided for this purpose, for example, on which environmental units can be traded. Such a marketplace can be formed centrally, or handled in each state or in a decentralized manner. For example, it could also be possible to transfer environmental units via email or messenger.

In one embodiment, each environmental account can have a starting value of environmental units. Such a starting value can be credited to the environmental account once, for example, such as upon initial application/registration or upon birth, for example, as a life credit. However, it is also possible that, for example, a government credits a yearly amount of environmental units to each user, so that in principle here a continuous availability of mobility is provided. Excess environmental units, i.e., environmental units which are not required or claimed in a year, can also remain available in following years on the environmental account, or they can be traded.

The allocation of environmental units can be coupled, for example, to legal requirements, for example, treaties under international law. In this manner, for example, consideration can be taken of global climate goals.

It can be provided that the use of such an environmental account is made compulsory on various levels, so that, for example, a government or a city management receives significant design freedom for environmental protection.

In principle, the transport means can comprise any form of movement, in particular driving, running, swimming, and/or flying. Accordingly, for example, motor vehicles, boats, and/or aircraft, but also bicycles, cargo bicycles, and/or drones can be used as transport means.

In particular, such a method can advantageously be used upon the use of autonomous vehicles, since they can automatically follow a specified transport route due to various environmental factors and deviations are only possible with difficulty.

The environmental units can be defined as desired. An environmental unit could, for example, correspond to a specific quantity of a specific pollutant, for example, carbon dioxide or nitrogen oxides. An environmental unit could, however, also comprise various components or could be a virtual dimension which represents environmental pollution per se in abstract form. For example, methane is significantly more harmful to the climate than carbon dioxide. It would therefore be possible to count a quantity unit of methane at higher costs of an environmental unit than, for example, the same quantity unit of carbon dioxide.

The invention also comprises a system for reducing the environmental pollution due to a transport route. The system is characterized according to the invention in that the system has an environmental account for managing environmental units, and that the system has a device for selecting a transport route, having a connection to the environmental account.

The selection of the transport route can take place on the basis of different criteria in this way. In contrast to the prior art, not only the shortest or fastest transport route can be considered in this case, but rather also one having the lowest environmental pollution. The selection of the transport means and/or a suitable route can also influence the environmental pollution here.

In one advantageous embodiment, the transport route can be selected at least according to the number of the environmental units available on the environmental account. In this way, it is possible to prevent a negative environmental balance from occurring.

In one embodiment, the system has a marketplace for trade, exchange, or other types of transfer of environmental units. Environmental units can thus be transferred between users.

In one embodiment, the system can be uniquely assigned to a transport means and/or transport material and/or user.

In one advantageous embodiment, the system is configured to ascertain the costs of a transport route in dependence on the actually covered transport route. For this purpose, the system can have means for detecting and/or monitoring the actual transport route. Such means can comprise, for example, a satellite navigation.

The system can be designed in particular to execute a method according to the invention as described above.

The invention is explained in greater detail hereinafter on the basis of the appended drawings.

In the figures:
- Figure 1:: shows a flow chart of a method according to the invention,
- Figure 2:: shows a block diagram of a system according to the invention, and
- Figure 3:: shows a schematic illustration of two alternative transport routes.

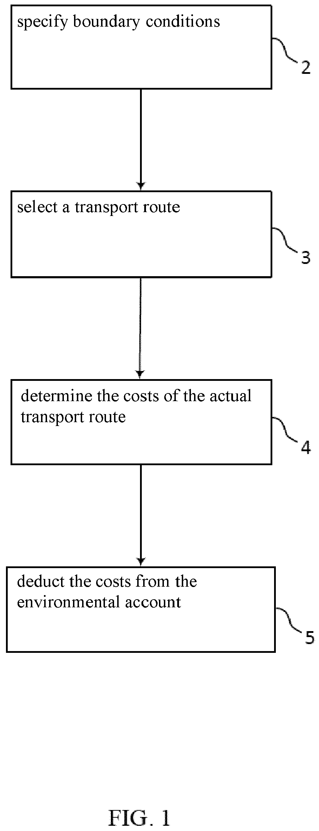

Figure 1 shows a flow chart of a method according to the invention. In a first step 2, the boundary conditions of the transport route are defined. These include a transport material, such as one or more persons and/or products and/or goods. Furthermore, it is necessary to specify a starting location and a destination location.

In a following step 3, a transport route is selected from these data. The selection therefore includes at least one transport means and one route. In particular, the transport route is selected so that a minimal environmental pollution results. In this case, legal requirements can be taken into consideration as well as voluntary specifications. In addition, the selection can also be carried out according to the environmental units present on the environmental account, so that the environmental account does not fall below zero.

It can also be advantageous here if the transport route is divided into partial transport routes to minimize the environmental pollution as a whole.

After the transport route has been executed or carried out, in a third step 4, the actual costs of the selected transport route are determined. Since it is possible to deviate from the transport route selected in step 2, it is important to determine the actually occurring cost to consider the actually carried out transport route. The actually occurring costs are then deducted from the environmental account in a last step 5.

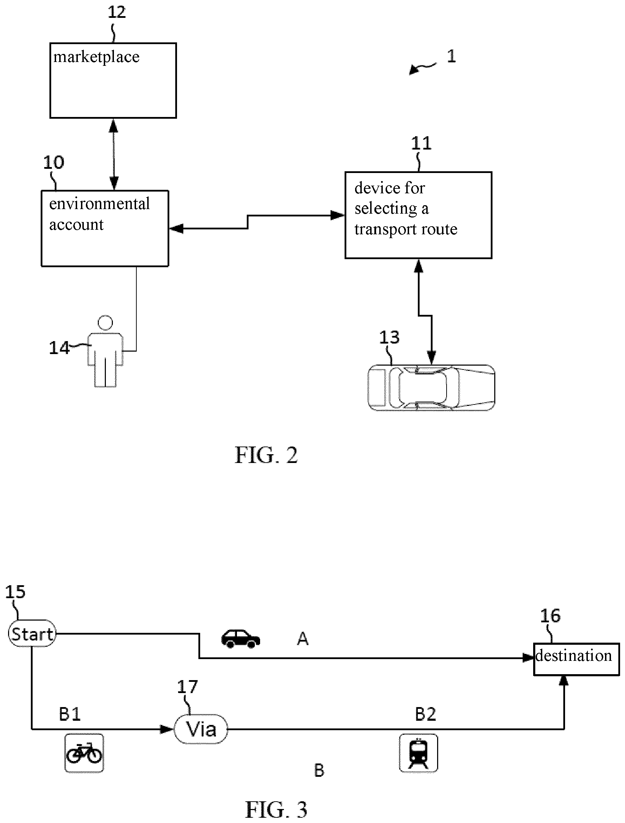

Figure 2 shows by way of example a system 1 according to the invention for reducing the environmental pollution. The system 1 has an environmental account 10, which is uniquely assigned to a person 14 in the example. Environmental units are managed on the environmental account 10. An environmental unit can be, for example, an abstract or virtual dimension which can represent various types of environmental pollution. For example, the various pollutants of a motor vehicle, such as CO2 and NOx, can be converted in weighted form into environmental units.

The system 1 has a device 11 for selecting a transport route. The transport route is selected in this case according to a method according to the invention to reduce or minimize the environmental pollution. In this case, the device 11 has a connection to the environmental account 10, whereby a consideration of the environmental units available on the environmental account 10 is possible.

The selected transport route can be transmitted, for example, to a transport means, in the example a motor vehicle 13, which was selected to carry out the transport route. The device 11 can be arranged, for example, in a portable device, it can be implemented solely as a software solution, for example, on a smart phone, or can be permanently installed in the vehicle 13. A unique assignment is possible at any time due to the connection to the environmental account 10. After ending the transport route, the environmental units actually required or consumed are deducted from the environmental account.

The system 1 can additionally have a marketplace 12, which is connected to the environmental account. Environmental units can be traded on the marketplace 12, to possibly purchase a missing number of environmental units which are required to carry out a certain transport route.

Two alternative transport routes A and B are shown by way of example in Figure 3, which are possible between a start 15 and a destination 16. For the first transport route A, a passenger vehicle was selected and a route which leads on the most direct possible path from the start 15 to the destination 16. The costs of this transport route are a certain number of environmental units.

However, it can be more favorable for minimizing the environmental pollution due to various factors to be considered, for example, to select the transport route B. In this case, for example, at typical commuting times, the costs of the transport route A can be made more expensive. Parts of the route A could also be blocked due to high environmental pollution or the environmental account could not have sufficient environmental units.

The transport route B is divided here into two partial transport routes Bland B2. The first partial transport route B1 leads with the bicycle to a railway station 17. From there, the second partial route B2 leads with the train to the destination 16. The environmental pollution of the transport route B is thus less as a whole than the environmental pollution of the transport route A.

However, the route selection shown is merely an example of how an environmentally-friendly route selection can take place. The example shown here are not meant to be restrictive in any way. All known and available transport means can be taken into consideration in the selection of the transport route.

### List of reference signs

- 1: system for reducing the environmental pollution
- 2-5: method steps
- 10: environmental account
- 11: device for selecting a transport route
- 12: marketplace
- 13: transport means
- 14: user
- 15: starting location
- 16: destination
- 17: railway station
- A: transport route
- B: transport route
- B1: partial transport route of the transport route B
- B2: partial transport route of the transport route B

## Claims

1. A method for reducing the environmental pollution due to a transport route, wherein the transport route contains at least one transport means, a transport material, and a route, **characterized in that**
an environmental account (10) is provided, on which environmental units are managed,
**in that** a transport route causes costs in the form of a number of environmental units and these costs are deducted from the environmental account, and
**in that** a transport route is selected in dependence on the costs.

2. The method as claimed in claim 1, **characterized in that** the environmental account is assigned to a transport means and/or a user and/or a transport material.

3. The method as claimed in one of claims 1 or 2, **characterized in that** the transport route is selected in dependence on the available environmental units on the environmental account (10), so that the environmental account does not sink below zero environmental units.

4. The method as claimed in any one of claims 1 to 3, **characterized in that** a transport route is selected in dependence on health data and/or fitness data of a user (13) and/or in dependence on an environmental pollution of a route.

5. The method as claimed in any one of claims 1 to 4, **characterized in that** a transport route is selected in dependence on administrative requirements and/or **in that** a transport route is selected in dependence on requirements which are voluntary or under private stipulations.

6. The method as claimed in any one of claims 1 to 5, **characterized in that** a transport route (B) is divided into multiple partial transport routes (B1, B2), wherein each partial transport route (B1, B2) has at least a transport means and a route and costs linked thereto in environmental units for the environmental account.

7. The method as claimed in any one of claims 1 to 6, **characterized in that** an ascertainment of the costs of a transport route is performed in dependence on the actually covered transport route.

8. The method as claimed in any one of claims 1 to 7, **characterized in that** the costs of a transport route are dependent on the transport means, on the route, on the current environmental pollution, on the utilization of a transport means, and/or on the transport material.

9. The method as claimed in any one of claims 1 to 8, **characterized in that** environmental units can be traded, given, loaned, or transferred in other ways, preferably on a marketplace.

10. The method as claimed in any one of claims 1 to 9, **characterized in that** each environmental account has a starting value of environmental units and/or **in that** environmental units are regularly credited to an environmental account.

11. The method as claimed in any one of claims 1 to 10, **characterized in that** the transport means can comprise any form of movement, in particular driving, running, swimming and/or **in that** the transport material can comprise persons and/or products and/or goods.

12. A system (1) for reducing the environmental pollution due to a transport route, wherein the transport route contains at least one transport means, a transport material, and a route, **characterized in that**
the system has an environmental account (10) for managing environmental units, and
**in that** the system has a device (11) for selecting a transport route, having a connection to the environmental account,
in particular wherein the transport route is selected at least according to the number of the environmental units available on the environmental account.

13. The system as claimed in claim 12, **characterized in that** the system has a marketplace (12) for trading, exchanging, or other types of transfer of environmental units.

14. The system as claimed in claim 12 or 13, **characterized in that** the device is uniquely assigned to a transport means and/or transport material and/or user (13).

15. The system as claimed in any one of claims 12 to 14, **characterized in that** the device for selecting a transport route is configured to ascertain the costs of a transport route in dependence on the actually covered transport route.
